(19) Europäisches Patentamt
European Patent Office
Office européen des brevets

(11) EP 2 965 090 B1

(12) EUROPEAN PATENT SPECIFICATION

(45) Date of publication and mention of the grant of the patent:
25.04.2018 Bulletin 2018/17

(21) Application number: 14726887.4

(22) Date of filing: 04.03.2014

(51) Int Cl.:
*G01N 33/68* (2006.01)

(86) International application number:
PCT/EP2014/054185

(87) International publication number:
WO 2014/135546 (12.09.2014 Gazette 2014/37)

(54) **METHODS AND COMPOSITIONS FOR THE DIAGNOSIS OF ALZHEIMER'S DISEASE**

VERFAHREN FÜR DIE DIAGNOSE VON ALZHEIMERSKRANKHEIT

MÉTHODES ET COMPOSITIONS POUR LE DIAGNOSTIC DE LA MALADIE D'ALZHEIMER

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR

(30) Priority: 05.03.2013 GB 201303936

(43) Date of publication of application:
13.01.2016 Bulletin 2016/02

(73) Proprietor: Randox Teoranta
Dungloe (IE)

(72) Inventors:
• FITZGERALD, Peter
Crumlin
Antrim BT29 4QY (GB)
• MCCONNELL, Ivan
Crumlin
Antrim BT29 4QY (GB)
• LAMONT, John
Crumlin
Antrim BT29 4QY (GB)
• RICHARDSON, Ciaran
Dungloe
Co. Donegal (IE)

(74) Representative: Gill Jennings & Every LLP
The Broadgate Tower
20 Primrose Street
London EC2A 2ES (GB)

(56) References cited:
EP-A2- 2 664 923          WO-A2-2006/134390
US-A1- 2007 099 203

• JERKOVIC L ET AL: "Afamin is a novel human vitamin E-binding glycoprotein characterization and in vitro expression", JOURNAL OF PROTEOME RESEARCH, AMERICAN CHEMICAL SOCIETY, WASHINGTON, DC, US, vol. 4, no. 3, 1 May 2005 (2005-05-01), pages 889-899, XP002371055, ISSN: 1535-3907, DOI: 10.1021/PR0500105

• ZHANG JING ET AL: "QUANTITATIVE PROTEOMICS OF CEREBROSPINAL FLUID FROM PATIENTS WITH ALZHEIMER DISEASE", JOURNAL OF ALZHEIMER'S DISEASE, IOS PRESS, NL, vol. 7, no. 2, 1 April 2005 (2005-04-01), page 125, XP009076593, ISSN: 1387-2877

• SONG F ET AL: "Screening plasma for biomarkers of mild cognitive impairment and Alzheimer's disease by quantitative proteomic methods", ALZHEIMER'S & DEMENTIA: THE JOURNAL OF THE ALZHEIMER'S ASSOCIATION, ELSEVIER, NEW YORK, NY, US, vol. 6, no. 4, 1 July 2010 (2010-07-01), pages S129-S130, XP027438934, ISSN: 1552-5260, DOI: 10.1016/J.JALZ.2010.05.406 [retrieved on 2010-07-01]

• John M Ringman ET AL: "Proteomic changes in cerebrospinal fluid of presymptomatic and affected persons carrying familial Alzheimer disease mutations", Archives of neurology, 1 January 2012 (2012-01-01), pages 96-104, XP55134102, United States DOI: 10.1001/archneurol.2011.642 Retrieved from the Internet: URL:http://www.ncbi.nlm.nih.gov/pubmed/22232349

- **PETRA ZÜRBIG ET AL: "Use of proteomic methods in the analysis of human body fluids in Alzheimer research", ELECTROPHORESIS, vol. 33, no. 24, 19 December 2012 (2012-12-19), pages 3617-3630, XP55134112, ISSN: 0173-0835, DOI: 10.1002/elps.201200360**

**Description**

**Background of the invention**

Alzheimer's disease (AD) is a chronic neurodegenerative disease currently identified by progressive cognitive impairment and loss of memory leading to severe dementia. AD is typically a disease of the elderly, most prevalent in persons over the age of 65. It is the leading cause of dementia in the elderly and with an increasingly higher life expectancy, the prevalence in the population is only set to increase. AD is not typically life threatening, however as the disease progresses to severe dementia, patients are unable to care for themselves and usually require full time professional care.

There is currently no known cure for AD, but there are treatments that can slow the progression of the disease. Therefore a method that can identify patients with AD and potentially monitor their response to treatment would be an invaluable assay (tool for clinicians).

Current methods of diagnosis of AD involve mental assessment (such as MMSE), CT/MRI, measurement of cerebrospinal fluid for specific Tau or Beta-amyloid isoforms known in the art to be associated with AD or genotyping for genetic risk factors such as *Apolipoprotein E4 (ApoE4 variant);* there are currently no clinically validated blood biomarkers of AD. Deficiencies of these methods can include a lack of specificity, they can be open to errors in interpretation, and may be highly invasive; generally a true diagnosis can only be made post mortem. There are currently no routinely used biomarker methods to assist the positive diagnosis for AD.

The pathogenesis of AD is not fully understood, but pathological investigations of patients revealed the presence of neurofibrillary tangles (caused by accumulation of Tau protein) and Beta-amyloid plaques. There is also widespread neuronal and synaptic loss, which is thought to underlie the reduced cognitive and mnemonic function. The formation of plaques has been shown to cause neurodegeneration, however the causes of plaque formation are unknown. Diagnostic tests that identify specific isoforms of these proteins have been the main focus in diagnostic assay development. However, the presence of these proteins may indicate that the disease has progressed past a therapeutically viable stage and therefore earlier risk markers may be more beneficial.

There have been several inventions describing methods for diagnosing AD using blood biomarkers, these include; EP 2293075 A2 and WO 2011/143597 A1. EP2293075 identified several markers expressed in blood platelets using 2-D gel electrophoresis, which were differentially expressed between AD and control patients. These included variants of proteins which may correspond to a genetic susceptibility to AD. A further invention was described by these inventors (EP2507638) in which protein biomarkers were combined in an algorithm along with genotyping to improve the diagnostic model. In this algorithm patients whom were ApoE4 positive were more likely to have AD, as were patients whom were ApoE4 negative, but expressed two copies of the wild-type glutathione S-transferase 1 Omega (wtGSTO) gene. In the context of this previous invention, wtGSTO was defined as any GSTO gene which did not contain the rs4825 mutation (which encodes an Aspartic acid instead of an Alanine at residue 140 [A140D]). This invention highlights the effectiveness of combining blood-based biomarkers and genotyping to assist in the diagnosis of disease. WO 2011/143597 A1 identified multiple biomarkers that are differentially expressed between serum of AD and control patients using multiplexed assays. In this invention, greater accuracy of diagnosis is observed when using multiple combinations of biomarkers combined with clinical measurements and demographic variables using Random forests to develop a classification algorithm. However, these methods have not found clinical utility and there is an urgent need for a method that can be used routinely to aid the diagnosis of AD.

**Summary of the Invention**

The present invention relates to methods and compositions for the diagnosis of Alzheimer's disease.

The present invention identifies and describes proteins that are differentially expressed in the Alzheimer's disease state relative to their expression in the normal state.

According to the first aspect of the invention, there is provided a method of diagnosing or monitoring a person at risk of developing Alzheimer's disease (AD), comprising measuring the concentration or relative levels of Afamin and Alpha-1 antichymotrypsin, and optionally at least one additional biomarker selected from Alpha-2-macroglobulin, Apolipoprotein B100, complement C3, Serine threonine kinase TBK-1, vitamin D binding protein, alpha-1-B glycoprotein, hemopexin, serum albumin, ceruloplasmin, alpha-2-antiplasmin, apolipoprotein A1, complement factor H, IgG, IgG fc binding protein, hornerin, fibrinogen or complement C5 in a fluid sample obtained from a person suspected of having or at least at risk of having AD, and establishing the significance of the concentrations or relative levels. Preferably the sample is serum or plasma.

In another embodiment, the relative levels of the differentially expressed proteins are used in conjunction with at least one of the ApoE or GSTO1 genotype or phenotype of a subject through identification of the nucleic acid sequence encoding the protein in the genome or through determining the form of protein produced in a fluid sample taken from the subject. This can increase the ability to differentiate between patients at risk of developing or having AD and those who are not at risk or do not have AD.

In some embodiments, the method for predicting the likelihood that a subject can be defined as suffering from or at risk of developing Alzheimer's disease, may comprise a categorical prediction model using statistical modelling or machine learning methods. Such methods may include, but are not limited to; perceptron neural networks, support vector machines, logistic regression, decision trees and random forests.

## Description of Drawings

Figures 1-9, boxplots comparing relative levels of Afamin (BSI0268), Afamin (BSI0223), Afamin (BSI0220), Alpha-1-antichymotrypsin (BSI0221), Complement C5 (BSI0782), Not known (BSI0183), Not known (BSI0279), Complement C3 (BSI0243) and Alpha-1B-glycoprotein (BSI0182) of control and AD patients.

Figure 10, ROC curve for use of Afamin (BSI0268) to discriminate between Control and AD Patients.

Figure 11, ROC curve for use of Alpha-1 antichymotrypsin (BSI0221) to discriminate between Control and AD Patients.

Figure 12, ROC curve for use of a model comprising Afamin (BSI0268) and Alpha-1 antichymotrypsin (BSI0221) to discriminate between Control and AD Patients.

Figure 13, ROC curve for use of the ratio of Afamin (BSI0268) and Alpha-1 antichymotrypsin (BSI0221) to discriminate between Control and AD Patients.

Figure 14, ROC curve for use of a model comprising Afamin (BSI0268)/Alpha-1 antichymotrypsin (BSI0221) ratio and Complement C3 (BSI0217) to discriminate between Control and AD Patients.

Figure 15, ROC curve for use of a model comprising Afamin (BSI0268)/Alpha-1 antichymotrypsin (BSI0221) ratio and Alpha-2 macroglobulin (BSI0195) to discriminate between Control and AD Patients.

Figure 16, ROC curve for use of a model comprising Afamin (BSI0268)/Alpha-1 antichymotrypsin (BSI0221) ratio and Serine threonine protein kinase TBK1 (BSI0112) to discriminate between Control and AD Patients.

Figure 17, ROC curve for use of a model comprising Afamin (BSI0268)/Alpha-1 antichymotrypsin (BSI0221) ratio and Complement C5 (BSI0792) to discriminate between Control and AD Patients.

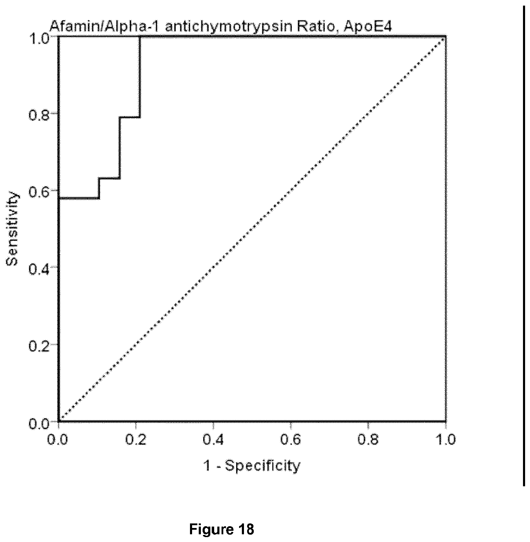

Figure 18, ROC curve for use of a model comprising Afamin (BSI0268)/Alpha-1 antichymotrypsin (BSI0221) ratio and ApoE4 status to discriminate between Control and AD Patients.

Figure 19, ROC curve for ability of ApoE4 status to discriminate between Control and AD Patients.

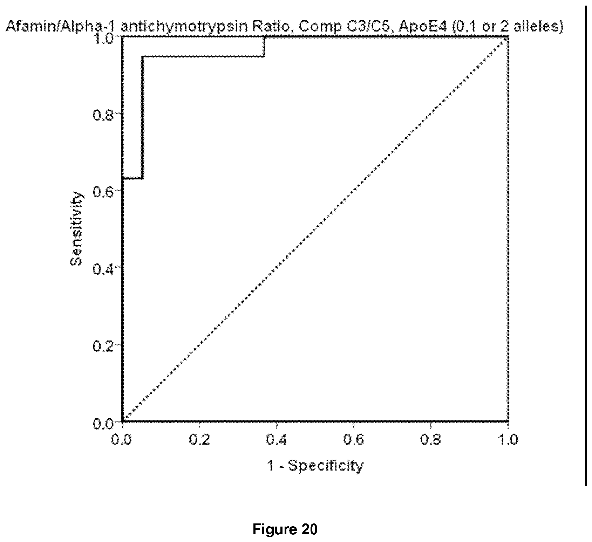

Figure 20, ROC curve for use of a model comprising Afamin (BSI0268)/Alpha-1 antichymotrypsin (BSI0221) ratio, Complement C5 (BSI0792) and ApoE4 status to discriminate between Control and AD Patients.

Figure 21, decision tree for use of Afamin/ Alpha-1 antichymotrypsin ratio and ApoE status to distinguish between Control and AD patients.

## Detailed description of the Invention

The present invention describes a biomarker-based method to aid in the diagnosis of Alzheimer's disease (AD). Specifically the measurement of relative levels or concentration of biomarkers within a fluid sample taken from a patient suspected of having or at risk of developing Alzheimer's disease are measured. The disclosure may also be used for monitoring the progression of AD and diagnosing and monitoring other forms of dementia and cognitive disorders, these include but are not limited to; Parkinson's dementia, Lewy body dementia, Vascular dementia, mild cognitive impairment, frontotemporal dementia. The term 'biomarker', in the context of the current invention, refers to a molecule present in a biological sample of a patient, the levels of which in said biological fluid may be indicative of Alzheimer's disease. Such molecules may include peptides/proteins or nucleic acids and derivatives thereof; the term 'relative levels', in the context of the current invention refers to the light intensity or absorbance reading (However the invention is not restricted to measurement using these techniques, the skilled person will be aware of other methods for measuring biological molecules that do not utilise measuring the properties of visible light to determine a measurement) from a biological assay that results from comparing the levels of the biomarker in a given biological sample to a reference material with a known concentration (this concentration may be zero) of the biomarker or level by which the biomarker within a biological sample directly competes with a reference material known to contain said biomarker to bind to a specific probe for said biomarker, the latter method generates a level inversely related to the concentration of the biomarker; the term 'probe' in the context of the current invention, refers to a synthetic or biological molecule that specifically binds to a region of a biomarker. The term 'at risk of developing Alzheimer's disease', in the context of the current invention, refers to a patient that displays early clinical signs; such as mild cognitive impairment (MCI) or vascular dementia determined by methods known in the art (*such as MMSE*), has family history of Alzheimer's disease, has genetic prevalence for Alzheimer's disease or is classified 'at risk' due to lifestyle (e.g. age, diet, general health, occupation, geographical location). The term 'genetic prevalence' in the context of the current invention, can imply that the patients genome contains specific genotypes for certain proteins which are known in the art to be altered in patients who develop AD, such proteins include, but are not limited to, Apolipoprotein E (ApoE) and Glutathione S-Transferase Omega 1 (GSTO). The genotype may be determined through genotyping or identifying the disease relevant form of the expressed protein in a biological fluid from the patient. More specifically, the number of alleles encoding ApoE4 and wild-type GSTO (wtGSTO) variants shall be determined. The term wtGSTO, in the context of the current invention, refers to any variant of GSTO that does not contain the rs4825 mutation in the genomic sequence, or an alanine to aspartic acid substitution at residue 140 of the protein sequence. The invention describes various biomarkers for use in diagnosing AD either alone or in combination with other diagnostic methods or as complementary biomarkers. A complementary biomarker in the current context implies a biomarker that can be used in conjunction with other biomarkers for AD.

A first aspect of the invention describes a method for diagnosing AD in a subject suspected of having, at risk of developing or of having AD which comprises measuring in an in vitro sample obtained from the subject, the relative level or concentration of the biomarkers Afamin and Alpha-1 antichymotrypsin and optionally one or more additional biomarkers selected from Table 1, and establishing the significance of the relative level(s) or concentration(s) of said biomarkers. The significance of the relative level or concentration is gauged by comparing said relative level or concentration to a control value for the specific biomarker. The control value is derived from determining the relative level or concentration of said biomarker in a biological sample taken from an individual(s) who does not have AD, as determined by clinical assessment. For Afamin, the relative level or concentration in a patient with AD is reduced compared with a control value. The invention utilises a method of employing a combination of Afamin and Alpha-1 antichymotrypsin, and optionally at least one additional biomarker selected from Alpha-2-macroglobulin, Apolipoprotein B100, complement C3, Serine threonine kinase TANK Binding Kinase-1 (TBK1), vitamin D binding protein, alpha-1-B glycoprotein, hemopexin, serum albumin, ceruloplasmin, alpha-2-antiplasmin, apolipoprotein A1, complement factor H, IgG, IgG fc binding protein, hornerin, fibrinogen or complement C5. In some embodiments, the invention uses a method whereby the relative level or concentration of Afamin is divided by the relative level or concentration of Alpha-1 antichymotrypsin to produce a ratio of Afamin/Alpha-1 antichymotrypsin. The term 'ratio' in the context of the current embodiment of the invention, relates to dividing the value of one biomarker by the other, this value should be the same for both biomarkers and can be represented as a weight or moles of biomarker in a given volume (concentration) or by a light intensity or absorbance level generated by means of an assay (relative level). A further embodiment of the invention utilises the value of the ratio of Afamin/Alpha-1 antichymotrypsin in combination with relative levels or concentration of one or more biomarkers chosen from Alpha-1 antichymotrypsin, Alpha-2-macroglobulin, Apolipoprotein B100, complement C3 , Serine threonine kinase TBK-1, vitamin D binding protein, alpha-1-B glycoprotein, hemopexin, serum albumin, ceruloplasmin, alpha-2-antiplasmin, apolipoprotein A1, complement factor H, IgG, IgG fc binding protein, hornerin, fibrinogen or complement C5. For example, a preferred combination is the ratio of Afamin/Alpha-1 antichymotrypsin in combination with the relative level or concentration of Complement C3. Another preferred combination of the invention is the ratio of Afamin/Alpha-1 antichymotrypsin in combination with the relative level or concentration of serine threonine kinase TBK-1.

In some embodiments, the invention is directed to the use of Afamin, and Alpha-1 antichymotrypsin, and optionally at least one additional biomarkers selected from Alpha-2-macroglobulin, Apolipoprotein B100, complement C3 , Serine threonine kinase TBK-1, vitamin D binding protein, alpha-1-B glycoprotein, hemopexin, serum albumin, ceruloplasmin, alpha-2-antiplasmin, apolipoprotein A1, complement factor H, IgG, IgG fc binding protein, hornerin, fibrinogen or complement C5 as complementary biomarkers of AD. As complementary biomarkers they may be used for AD diagnosis in conjunction with other clinical evidence such as mental state assessment (MMSE), neurological imaging, Beta-amyloid peptides, phosphorylated Tau, ApoE genotype, and wild-type GSTO 1 genotype (wtGSTO). This clinical evidence may be added to the predictive model, based on the output measurement. For example, ApoE status of a patient may be determined through genotyping, by identifying the disease relevant form of protein that is expressed at the genetic level (DNA and/or RNA), or by detecting the presence of the specific expressed form of the protein from a fluid sample taken from the patient. This output may be expressed as either a dichotomised value, whereby the patient is either positive for the *ApoE4* gene or protein or not; or as an ordinal output for the number of ApoE4 alleles present in the patients genomic DNA (0-2), which can be calculated using relative levels of the gene or protein within a sample taken from the patient.

Biomarker relative levels or concentrations can be determined by contacting the sample with probes, preferably immobilised on a substrate, specific for each of the biomarkers included in the combination of biomarkers. Interactions between biomarker and its respective probe can be monitored and quantified using various techniques that are well-known in the art. An example of a suitable technique is an enzyme-linked immunosorbent assay (ELISA). Performing an ELISA involves at least one antibody with specificity for a particular antigen. The sample with an unknown amount of antigen is immobilized on a solid support (usually a polystyrene microtiter plate) either non-specifically (via adsorption to the surface) or specifically (via capture by another antibody specific to the same antigen, in a "sandwich" ELISA). After the antigen is immobilized, the detection antibody is added, forming a complex with the antigen. The detection antibody can be covalently linked to an enzyme, or can itself be detected by a secondary antibody that is linked to an enzyme through bioconjugation. Between each step, the plate is typically washed to remove any proteins or antibodies that are not specifically bound. After the final wash step, the plate is developed by adding an enzymatic substrate to produce a visible signal, which indicates the quantity of antigen in the sample.

In a preferred embodiment of the current invention the 'sample' as referred to herein is serum or plasma, however it may be any sample from a patient from which biomarker levels or concentrations can be determined. These include but are not limited to whole blood, urine, saliva, cerebrospinal fluid and platelets.

The substrate will comprise probes specific to the Afamin and Alpha-1 antichymotrypsin biomarkers. As used herein, the term 'specific' means that the probe binds only to one of the biomarkers of the invention, with negligible binding to other biomarkers of the invention or to other analytes in the biological sample being analysed. This ensures that the integrity of the diagnostic assay and its result using the biomarkers of the invention is not compromised by additional binding events.

The substrate can be any surface able to support one or more probes, but is preferably a biochip. A "Biochip" is a general term for a reaction platform for hosting chemical, biochemical, proteomic or molecular tests, as may be required for medical diagnosis, drug detection, etc. Typically, a Biochip comprises an inert substrate, such as silicon, glass or ceramic (often of the order of about 1 cm$^2$ or less in surface area), on which one or a plurality of reaction sites is provided. The sites generally carry one or more ligands, for example, one or more antibodies, selected for the test (or "assay") to be performed, adsorbed to the surface of the chip for activation upon combination with a sample applied to the chip (e.g. a blood sample) and/or a reagent. The reactions can be detected using a number of alternative techniques, including detection of chemiluminescence generated by the reaction. Some biochips carry a very large number (hundreds or thousands) of such tests sites, typically arranged in a grid or array, making it possible to carry out numerous assays simultaneously, and using the same single specimen. When identifying the various biomarkers/proteins of the invention it will be apparent to the skilled person that as well as identifying the full length protein, the identification of a fragment or several fragments of a protein is possible, provided this allows accurate identification of the protein. Similarly, although a preferred probe of the invention is a monoclonal antibody, other probes such as aptamers, molecular imprinted polymers, phages, short chain antibody fragments and other antibody-based probes may be used. The invention also allows for nucleic acid sequence probes.

Preferably, a solid state device is used in the methods of the present invention, preferably the Biochip Array Technology system (BAT) (available from Randox Laboratories Limited). More preferably, the Evidence Evolution and Evidence Investigator apparatus (available from Randox Laboratories) may be used to determine the levels of biomarkers in the sample.

The accuracy of statistical methods used in accordance with the present invention can be best described by their receiver operating characteristics (ROC). The ROC curve addresses both the sensitivity, the number of true positives, and the specificity, the number of true negatives, of the test. Therefore, sensitivity and specificity values for a given combination of biomarkers are an indication of the accuracy of the assay. For example, if a biomarker combination has sensitivity and specificity value of 80%, out of 100 patients, 80 will be correctly identified from the determination of the presence of the particular combination of biomarkers as positive for disease, while out of 100 patients who do not have disease 80 will accurately test negative for the disease.

If two or more biomarkers are to be used in the diagnostic method a suitable mathematical or machine learning classification model, such as logistic regression equation, can be derived. The logistic regression equation might include other variables such as age and gender of the patient. The ROC curve can be used to assess the accuracy of the logistic regression model. The logistic regression equation can be used independently or in an algorithm to aid clinical decision making. Although a logistic regression equation is a common mathematical/statistical procedure used in such cases and is preferred in the context of the present invention, other mathematical/statistical, decision trees or machine learning procedures can also be used.

By way of example, a logistic regression equation applicable to the present invention (at a classification cut-off value of 0.5) for the biomarker combination for indication of AD versus non-AD (control) in a patient suspected of having or being at risk of developing AD is calculated as follows;

$$Probability\ of\ AD = \frac{1}{1 + e^{-\left(3.1+9.4\left[\frac{Albumin}{Alpha\ 1\ antichymotrypsin}\right]-22.6[Complement\ C5]\right)}}$$

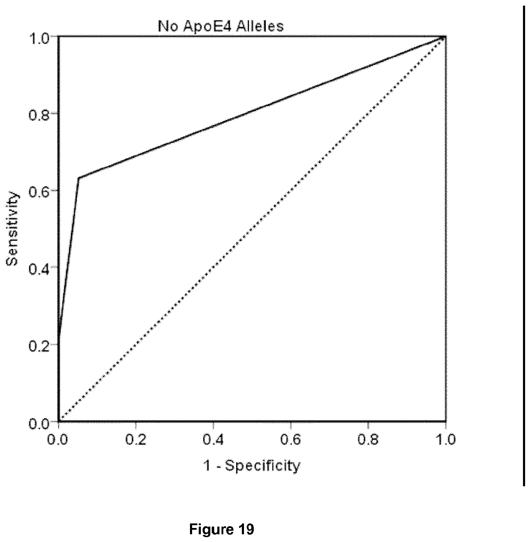

As further example, a decision tree may be grown where a decision branch is grown from each node (sub-population) to divide the population into classification groups. Figure 19 represents an example of a tree that was grown using the data described in this invention, which could correctly classify all AD patients with a relatively small error.

**Methods**

1. Normalised plasma/Quantiplasma™

Plasma normalisation was conducted as per US 2009/0136966. Briefly human plasma was normalised by removing high abundance proteins utilising the propriety method. Firstly, high abundance proteins were removed using Multiple Affinity Removal System (MARS) technology. The resultant plasma was then loaded on to a Multi-ImmunoAffinity Normalisation (MIAN) column, where normalisation stringency was adjusted by altering the flow rate. The flow-throw and wash samples were combined to give a differentially normalised sample. Some of this normalised plasma was then ubiquitously biotinylated to provide a tracer substance, known as Quantiplasma™.

2. Antibody generation

Monoclonal antibodies were produced as per US 2009/0136966. Normalised plasma was used as an immunogen to generate polyclonal antibodies. B-cells were then isolated and monoclonal hybridomas were generated. Initial selection of hybridomas was done using an ELISA. Plates were coated with mouse Ig gamma-Fc specific GAM, and then incubated with the mAb hybridoma supernatant, following a wash step this complex was then incubated with the Quantiplasma™ and finally an enzyme-substrate reaction was induced to detect the binding of the biotinylated plasma (Quantiplasma™) to the mAb. This selection identified more than 1000 mAb. To identify the protein targets of monoclonal antibodies used in this study, western blotting, immunoprecipitation and mass spectrometry techniques were employed. There are, however, some antigens that could not be identified at this time, but as they are known to be present in the human plasma proteome they have been included.

3. Identification of clinical biomarkers using Quantiplasma™

Serum samples were obtained from 19 clinically confirmed Alzheimer's disease (AD) patients and 19 age/gender-matched control participants with normal cognitive function. These samples were frozen shortly after collection and stored at (-80°C) until analysis was performed. Additional clinical information was gathered for these subjects, this included basic personal and family medical history. Further to this, ApoE and GSTO genotype were determined through methods known in the art. For each patient, genomic DNA was isolated and the presence of DNA that encodes each of the 3 isoforms of ApoE (E2, E3, E4) or GSTO (wild-type, mutant A140 [rs4825]) were determined utilising polymerase chain reaction (PCR) techniques. Further analysis allowed the allelic frequency of each of the isoforms to be determined through methods known in the art.

A panel of 69 mAb antibodies (Table 1) were selected out of a catalogue of >1000 generated as per Section 2. Antibodies were then evaluated by competitive immunoassay. They were first immobilised on a biochip platform (9mm x 9mm), which was the substrate for the immunoreactions. The semi-automated bench top analyser Evidence Investigator was used (EV3602, Randox Laboratories Ltd., Crumlin, UK, patents-EP98307706, EP98307732, EP0902394, EP1227311, EP1434995 and EP1354623). The assay principle is based on competition for binding sites of the monoclonal antibody between free antigen (the patient sample) and labelled tracer plasma (Quantiplasma™).

Sample and reagents are added to the biochip and incubated under controlled conditions. Following addition of substrate, a light signal is generated which is then detected using digital imaging technology. The system incorporates dedicated software to automatically process, report and archive the data generated. The level of a specific protein in the patient sample is determined by comparing the difference between the light signal (RLU) at the position of the respective antibody on a biochip containing sample and the tracer (test) and a biochip containing just the tracer (blank). A ratio between test and control samples is determined as;

$$\text{relative level of biomarker} = 1 - \frac{RLU(test)}{RLU(blank)}$$

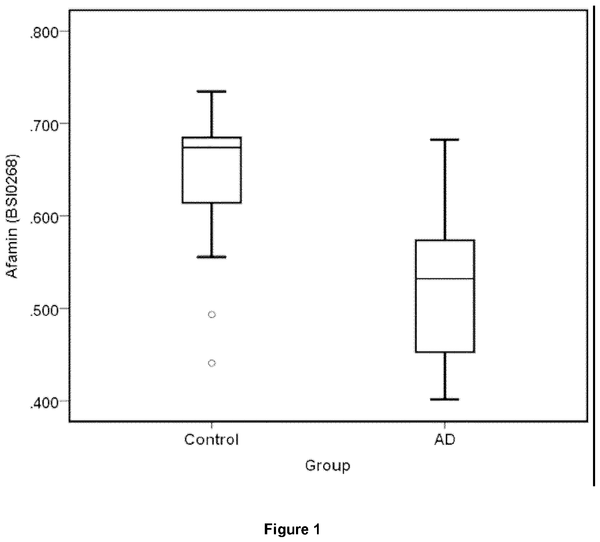

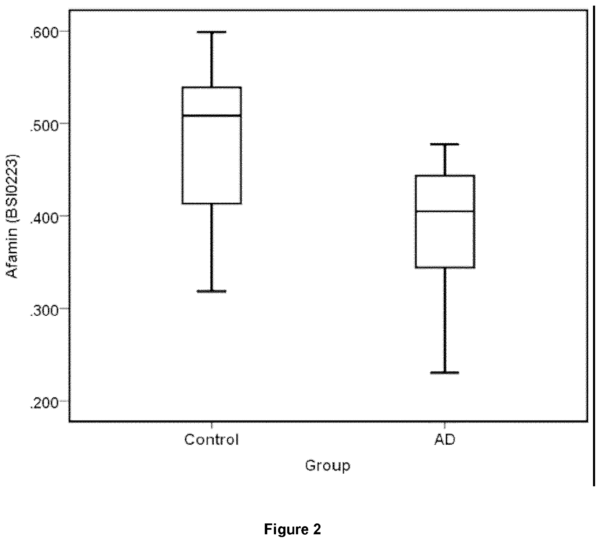

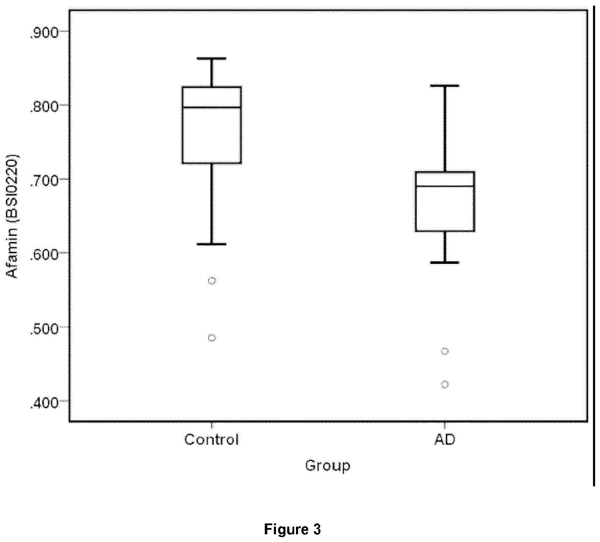

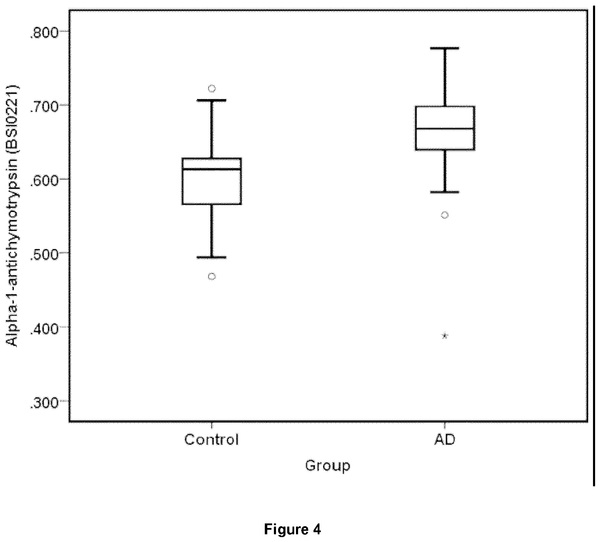

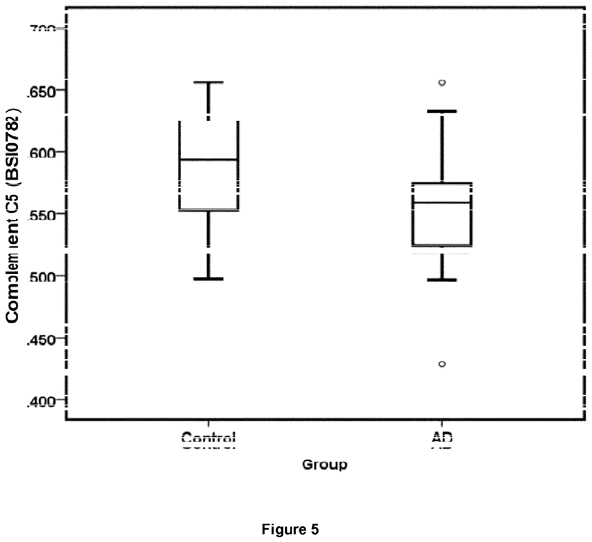

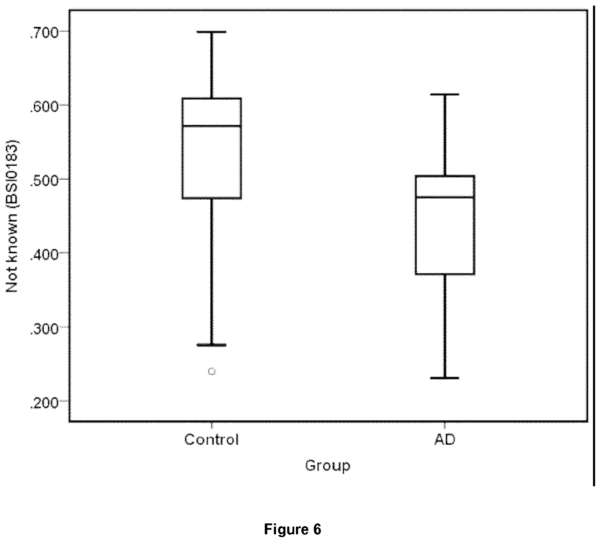

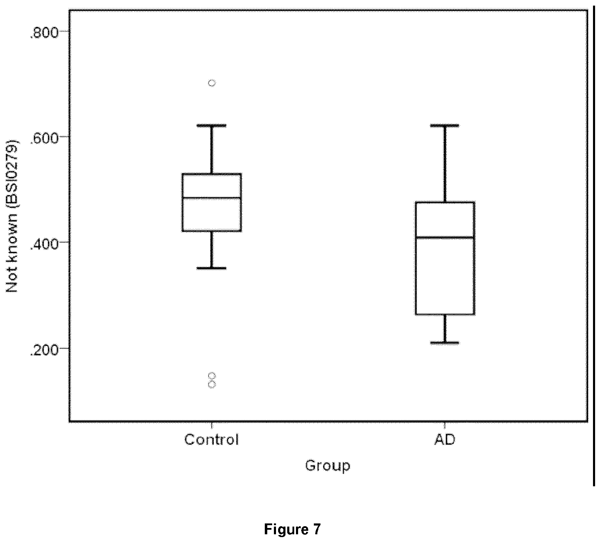

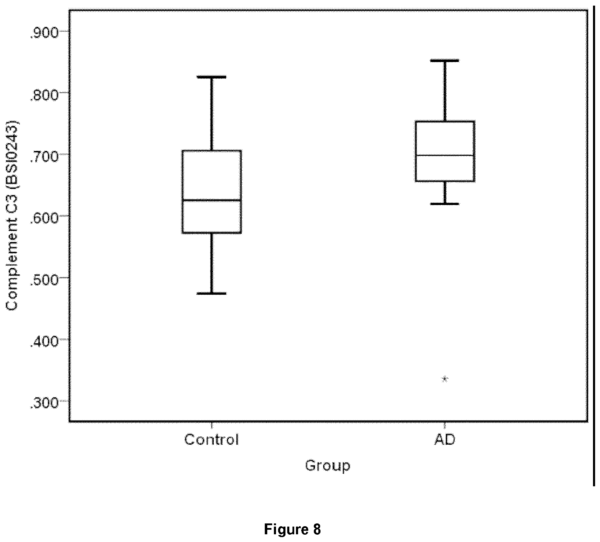

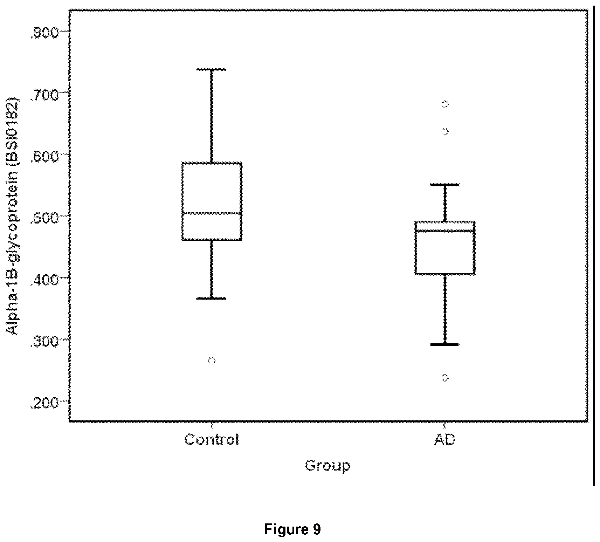

with a high ratio indicating a relatively high level of the protein specific for its respective mAb present in the sample, and a low ratio indicating relatively little or none of the protein present in the sample. Ratios for AD patients and control patients for all mAbs were determined (Example Figures 1-9) and non-parametric analysis was conducted to identify those mAbs which could distinguish between AD and control patients (Table 2). Areas under the curve (AUC) of the receiver operator characteristic (ROC) curves were calculated for all mAbs, these are detailed in Table 3.

4. Disease classification model

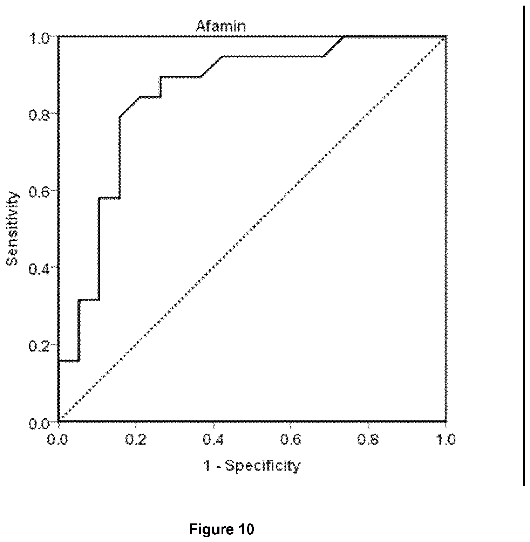

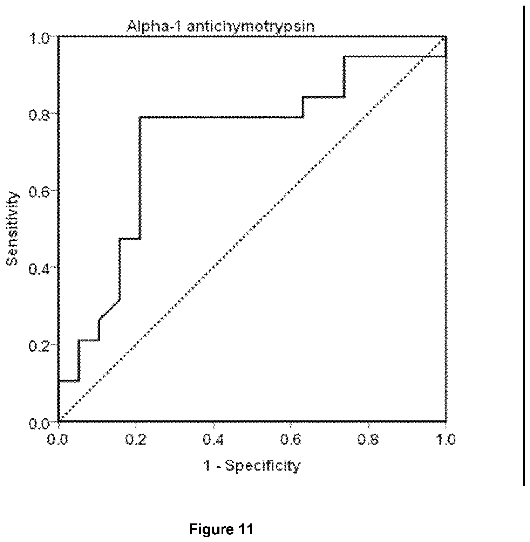

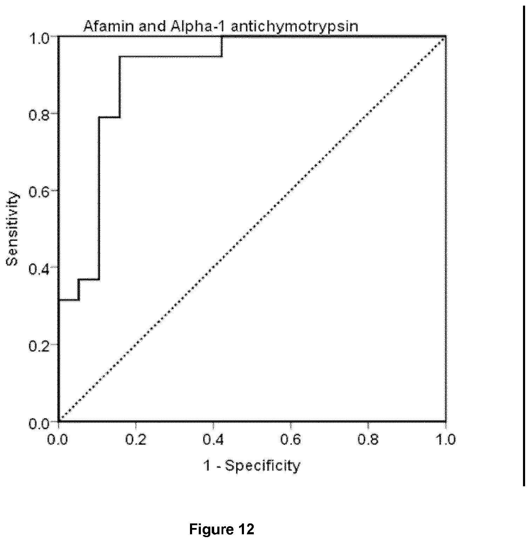

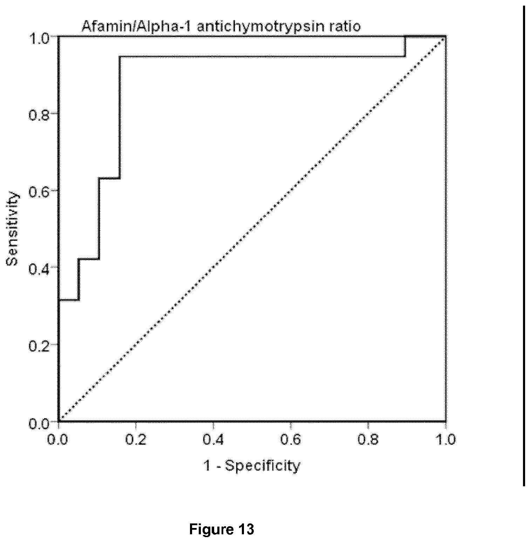

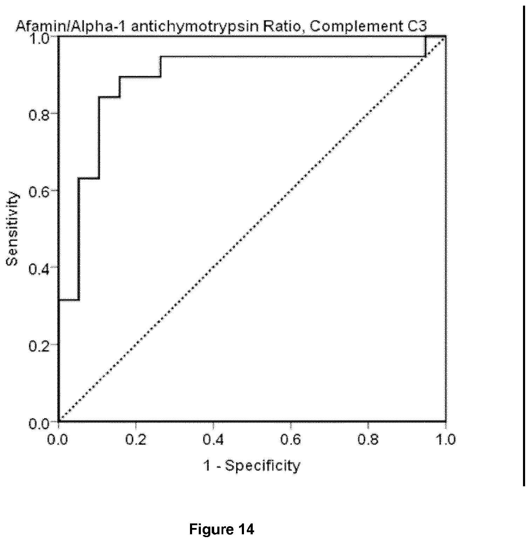

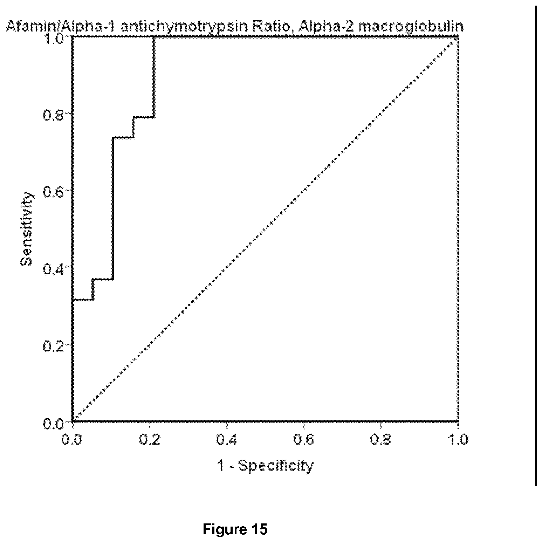

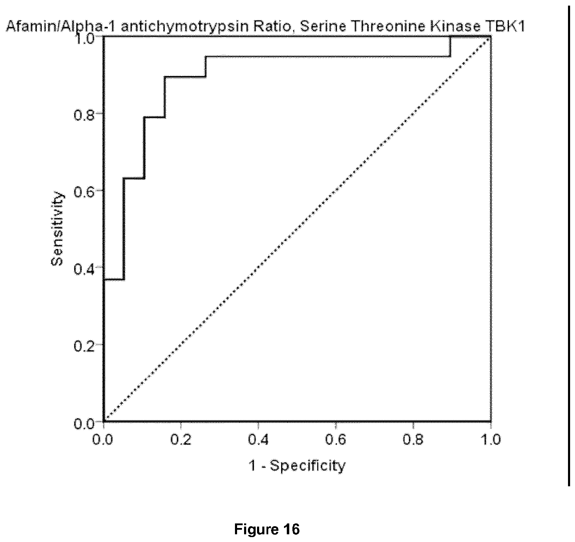

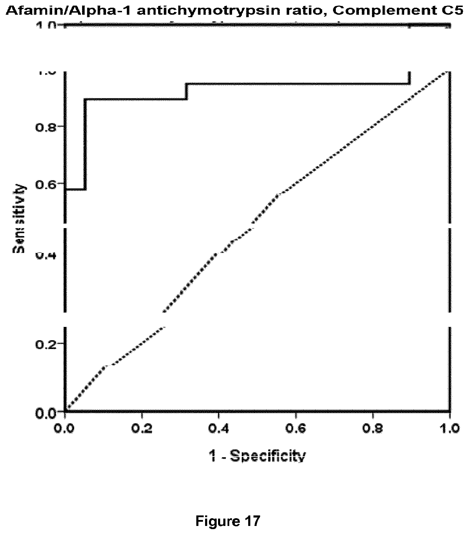

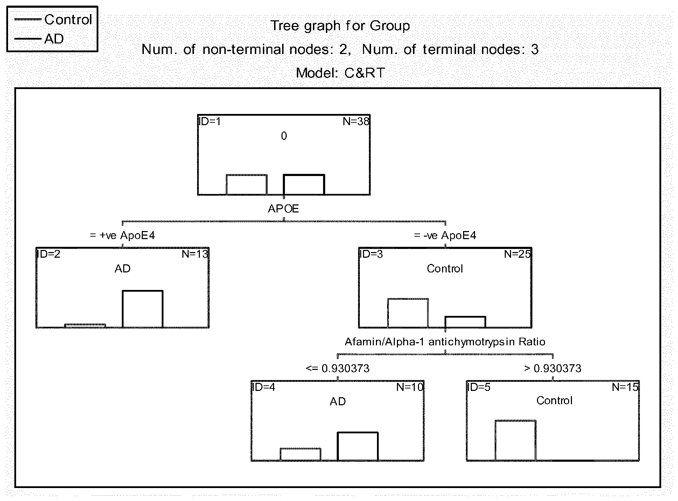

As an example of how multiple markers identified by this study may be combined to provide a model to classify a patient whose disease state is unknown, we have used logistic regression as a method of model determination. Initial investigation showed that using the relative levels of Afamin (BSI0268) combined with that of Alpha-1-antichymotrypsin (BSI0221) generated a model with an AUC of 0.906 (Fig 10-13), a significant improvement on the predictive power of the individual measurements. In order to add further analytes to the model, without increasing the dimensions, a function of Afamin (BSI0268) as a proportion of Alpha-1-antichymotrypsin (BSI0221) was used as a single variant (AUC=0.875) and the effect of adding all other analytes systematically in to the model was analysed. The addition of Complement C3 (BSI0217), Alpha-2 macroglobulin (BSI0195), Serine threonine protein kinase TBK1 (BSI0112) or Complement C5 (BSI0782) to the model improved the model, AUC of 0.889, 0.906, 0.892 and 0.920 respectively (Fig 14-17). Further, improvements were identified when considering the ApoE genotype of the patients. A categorical variable, whereby each patient was identified as having either no *Apoe4* alleles (0) or having one or more (1), was added to the analysis. This was further refined by identifying the number of *ApoE4* alleles each patient had (0, 1 or 2). In this study, 63% of the AD patients had at least one *Apoe4* allele, where only one out of the 19 control subjects (5%) was *Apoe4* positive. ApoE4 genotype in combination with the Afamin/Alpha-1 antichymotrypsin ratio produces an AUC of 0.925, this increases to 0.953 when taking into consideration the number of ApoE4 alleles. Furthermore, the AUC increases to 0.964 with the addition of Complement C5. These data suggest that a very accurate model may be generated using the current invention. These data are summarised in Table 4. The curse of dimensionality limits the number of variables that may be used in developing a model using this preliminary data set, but it is predicted that several markers included in this study may be combined to provide an optimal model. As well as logistic regression, other supervised learning models were generated using this data, such as; multi-layer perceptron neural networks, random forests, support vector machines and decision trees (Figure 21). These all provided models with similar accuracy as logistic regression and may be preferred as new analytes are added to the model.

*Table 1* mAb ID numbers and the respective protein that it has been found to bind to

| Probe ID | Protein ID |
|---|---|
| BSI0183 | Not known |
| BSI0185 | D vitamin binding protein |
| BSI0189 | Complement C3b (shorter form) |
| BSI0198 | Alpha-1-B glycoprotein |
| BSI0200 | Alpha-2-macroglobulin |
| BSI0203 | Ceruloplasmin |
| BSI0208 | Apo B100 |
| BSI0220 | Afamin |
| BSI0221 | Alpha-1-antichymotrypsin |
| BSI0197 | Alpha-2-macroglobulin |
| BSI0201 | Not known |
| BSI0214 | ApoB100 |
| BSI0190 | Complement C3 |
| BSI0191 | Not known |

(continued)

| Probe ID | Protein ID |
|----------|-----------|
| BSI0195 | Alpha-2-macroglobulin |
| BSI0223 | Afamin |
| BS10186 | Alpha-1-antichymotrypsin |
| BS10196 | Alpha-1B-glycoprotein |
| BSI0279 | Not known |
| BSI0281 | Not known |
| BSI0217 | Complement C3 |
| BSI0289 | ApoB100 |
| BSI0311 | Not known |
| BSI0144 | Alpha-1-antichymotrypsin |
| BSI0112 | Serine threonine protein kinase TBK1 |
| BSI0022 | Not known |
| BSI0002 | Hemopexin |
| BSI0032 | IgG Fc Binding protein |
| BSI0038 | Alpha-2-macroglobulin |
| BSI0023 | Not known |
| BSI0051 | D-vitamin binding protein |
| BSI0095 | Alpha-2-macroglobulin |
| BSI0097 | Serum albumin |
| BSI0116 | Alpha-1B-glycoprotein |
| BSI0099 | Ceruloplasmin |
| BSI0136 | ApoB100 |
| BSI0172 | Alpha-2-macroglobulin |
| BSI0177 | Not known |
| BSI0142 | Alpha-2-antiplasmin |
| BSI0173 | Alpha-2-macroglobulin |
| BSI0179 | Apolipoprotein A1 |
| BSI0180 | Not known |
| BSI0181 | Not known |
| BSI0100 | Serine threonine protein kinase TBK1 |
| BSI0182 | Alpha-1B-glycoprotein |
| BSI0040 | ApoB100 |
| BSI0314 | Not known |
| BSI0243 | Complement C3 |
| BSI0348 | Not known |
| BSI0257 | D-vitamin binding protein |
| BSI0263 | Complement C3 |
| BSI0268 | Afamin |

(continued)

| Probe ID | Protein ID |
|---|---|
| BSI0355 | Not known |
| BSI0660 | Not known |
| BSI0670 | ApoB100 |
| BSI0747 | Factor H |
| BSI0765 | Complement C5 |
| BSI0782 | Complement C5 |
| BSI0225 | Not known (IgG) |
| BSI0239 | Apolipoprotein A1 |
| BSI0242 | Complement C3 |
| BSI0246 | Not known |
| BSI0248 | IgG |
| BSI0255 | Hornerin |
| BSI0259 | Not known |
| BSI0266 | ApoB100 |
| BSI0323 | ApoB100 |
| BSI0115 | Alpha-2-antiplasmin |
| BSI0251 | Fibrinogen |

*Table 2 Summary statistics for all 69 mAbs for both Control (19) and AD (19) groups, p-value represents the significant difference between each group as determined by Mann-Whitney*

| Protein ID (mAb ID) | Control | | | AD | | | |
|---|---|---|---|---|---|---|---|
| | Median | Min | Max | Median | Min | Max | p-value |
| Afamin (BSI0268) | .674 | .441 | .735 | .532 | .402 | .683 | .00021 |
| Afamin (BSI0223) | .508 | .319 | .599 | .405 | .230 | .477 | .00384 |
| Afamin (BSI0220) | .796 | .485 | .863 | .690 | .422 | .826 | .00506 |
| Alpha-1-antichymotrypsin (BSI0221) | .613 | .468 | .722 | .668 | .388 | .777 | .01734 |
| Complement C5 (BSI0782) | .592 | .497 | .656 | .559 | .429 | .656 | .02648 |
| Not known (BSI0183) | .571 | .240 | .699 | .475 | .231 | .614 | .02853 |
| Not known (BSI0279) | .484 | .132 | .702 | .409 | .210 | .621 | .03304 |
| Complement C3 (BSI0243) | .625 | .474 | .826 | .698 | .336 | .852 | .04245 |
| Alpha-1B-glycoprotein (BSI0182) | .504 | .265 | .737 | .475 | .238 | .681 | .05396 |
| Alpha-1-antichymotrypsin (BSI0144) | .605 | .342 | .724 | .652 | .511 | .744 | .05584 |
| ApoB100 (BSI0289) | .025 | -1.623 | .779 | .291 | -1.089 | .819 | .05969 |
| Alpha-2-antiplasmin (BSI0115) | .302 | -.298 | .710 | .209 | -.131 | .562 | .07488 |
| Factor H (BSI0747) | .409 | .179 | .535 | .336 | .083 | .600 | .08493 |

(continued)

| Protein ID (mAb ID) | Control | | | AD | | | |
|---|---|---|---|---|---|---|---|
| | Median | Min | Max | Median | Min | Max | p-value |
| Serum albumin (BSI0097) | .725 | .643 | .756 | .701 | .583 | .772 | .08766 |
| Apolipoprotein A1 (BSI0239) | .411 | .275 | .522 | .382 | .250 | .478 | .11827 |
| Complement C3 (BSI0263) | .545 | -.166 | .915 | .143 | -.826 | .850 | .12534 |
| Alpha-2-macroglobulin (BSI0197) | .544 | .168 | .670 | .444 | .101 | .660 | .15249 |
| Complement C5 (BSI0765) | .309 | .131 | .947 | .263 | -.144 | .865 | .15254 |
| ApoB100 (BSI0040) | .155 | -.125 | .560 | .261 | -.136 | .697 | .15666 |
| Alpha-1B-glycoprotein (BSI0116) | .259 | .109 | .348 | .227 | .092 | .335 | .15679 |
| Not known (BSI0023) | .481 | .202 | .825 | .400 | .028 | .884 | .16104 |
| ApoB100 (BSI0670) | .644 | .420 | .776 | .626 | .408 | .735 | .16995 |
| Serine threonine protein kinase TBK1 (BSI0112) | .576 | -.207 | .691 | .464 | -.275 | .660 | .17460 |
| Not known (BSI0246) | .184 | .031 | .316 | .136 | -.006 | .295 | .17460 |
| Not known (BSI0348) | .687 | .416 | .806 | .646 | .350 | .781 | .17924 |
| Alpha-1-antichymotrypsin (BSI0186) | .478 | -.195 | .868 | .365 | -.691 | .736 | .18406 |
| Complement C3 (BSI0217) | .141 | -1.116 | .788 | .056 | -.361 | .716 | .19389 |
| ApoB100 (BSI0323) | .446 | .236 | .685 | .394 | .139 | .607 | .20409 |
| Alpha-1B-glycoprotein (BSI0196) | .431 | .239 | .549 | .453 | .255 | .569 | .21469 |
| Alpha-2-antiplasmin (BSI0142) | .643 | .454 | .712 | .663 | .585 | .728 | .21469 |
| Ceruloplasmin (BSI0203) | .367 | -.951 | .865 | .278 | -1.956 | .727 | .22567 |
| Fibrinogen (BSI0251) | .457 | .222 | .717 | .426 | .211 | .608 | .24281 |
| Not known (BSI0022) | .530 | .331 | .895 | .492 | .256 | .898 | .26718 |
| Alpha-1-B glycoprotein (BSI0198) | .723 | .611 | .791 | .697 | .376 | .771 | .29317 |
| Alpha-2-macroglobulin (BSI0172) | .759 | .642 | .865 | .737 | .678 | .887 | .30001 |
| D-vitamin binding protein (BSI0257) | .614 | .014 | .788 | .601 | .448 | .722 | .32087 |
| Complement C3 (BSI0190) | .454 | .268 | .621 | .513 | .294 | .646 | .34271 |
| Alpha-2-macroglobulin (BSI0038) | .636 | .331 | .738 | .575 | .246 | .748 | .35016 |
| Not known (BSI0201) | .711 | .495 | .818 | .688 | .490 | .867 | .35776 |
| Alpha-2-macroglobulin (BSI0195) | .940 | .796 | .974 | .924 | .423 | .982 | .37323 |
| Apolipoprotein A1 (BSI0179) | .794 | .328 | .917 | .761 | .110 | .937 | .37323 |

(continued)

| Protein ID (mAb ID) | Control | | | AD | | | |
| --- | --- | --- | --- | --- | --- | --- | --- |
| | Median | Min | Max | Median | Min | Max | p-value |
| ApoB100 (BSI0136) | .483 | .155 | .670 | .522 | .206 | .637 | .38910 |
| Complement C3b (shorter form) (BSI0189) | .607 | .487 | .839 | .585 | .495 | .747 | .39711 |
| Not known (BSI0660) | .945 | -.223 | .975 | .947 | -2.124 | .968 | .40535 |
| Apo B100 (BSI0208) | .572 | .202 | .694 | .595 | -.250 | .911 | .40538 |
| IgG (BSI0248) | .638 | .400 | .781 | .556 | .253 | .699 | .44774 |
| Hemopexin (BSI0002) | .567 | -2.587 | .830 | .708 | -1.046 | .890 | .49266 |
| Alpha-2-macroglobulin (BSI0095) | .761 | .592 | .924 | .689 | .645 | .871 | .49266 |
| Not known (BSI0281) | .744 | .511 | .799 | .740 | .502 | .837 | .50189 |
| Not known (BSI0355) | .423 | .254 | .686 | .431 | .097 | .602 | .51126 |
| Not known (more candidates) (BSI0177) | .108 | -.076 | .216 | .114 | -.051 | .296 | .55927 |
| Hornerin (BSI0255) | .513 | .255 | .824 | .487 | .146 | .920 | .61962 |
| Alpha-2-macroglobulin (BSI0173) | .692 | .482 | .836 | .661 | .567 | .833 | .63001 |
| D vitamin binding protein (BSI0185) | .613 | .294 | .743 | .606 | .381 | .782 | .65090 |
| Not known (IgG) (BSI0225) | .390 | .080 | .651 | .402 | .054 | .577 | .65090 |
| Not known (BSI0314) | .633 | .014 | .830 | .625 | .446 | .764 | .67206 |
| ApoB100 (BSI0214) | .401 | .073 | .675 | .386 | -.090 | .666 | .69349 |
| Not known (BSI0259) | .342 | .047 | .619 | .307 | .133 | .507 | .69349 |
| Ceruloplasmin (BSI0099) | .590 | .472 | .734 | .593 | .321 | .721 | .77029 |
| Not known (BSI0311) | .344 | .020 | .506 | .351 | -.031 | .601 | .82668 |
| Complement C3 (BSI0242) | .446 | .148 | .653 | .409 | .277 | .568 | .82668 |
| Not known (BSI0180) | .237 | .032 | .443 | .210 | .085 | .521 | .84949 |
| ApoB100 (BSI0266) | .528 | .303 | .704 | .516 | .216 | .648 | .84949 |
| D-vitamin binding protein (BSI0051) | .450 | .035 | .602 | .472 | .324 | .629 | .89548 |
| Not known (BSI0181) | .502 | .350 | .708 | .556 | .227 | .926 | .90702 |
| IgG Fc Binding protein (BSI0032) | .432 | -1.988 | .826 | .431 | -2.053 | .928 | .94182 |
| Alpha-2-macroglobulin (BSI0200) | .577 | .338 | .660 | .544 | .309 | .703 | .96507 |
| Serine threonine protein kinase TBK1 (BSI0100) | .767 | .174 | .839 | .768 | .680 | .815 | .96507 |
| Not known (BSI0191) | .542 | .328 | .898 | .583 | .285 | .902 | 1.00000 |

Table 3 AUC for the ROC curve for each of the 69 mAb for distinguishing Control from AD

| Protein ID (Probe) | AUC |
|---|---|
| Afamin (BSI0268) | .852 |
| Afamin (BSI0223) | .774 |
| Afamin (BSI0220) | .766 |
| Alpha-1-antichymotrypsin (BSI0221) | .726 |
| Complement C5 (BSI0782) | .711 |
| Not known (BSI0183) | .708 |
| Not known (BSI0279) | .702 |
| Complement C3 (BSI0243) | .693 |
| Alpha-1B-glycoprotein (BSI0182) | .683 |
| Alpha-1-antichymotrypsin (BSI0144) | .681 |
| ApoB100 (BSI0289) | .679 |
| Alpha-2-antiplasmin (BSI0115) | .669 |
| Factor H (BSI0747) | .663 |
| Serum albumin (BSI0097) | .662 |
| Apolipoprotein A1 (BSI0239) | .648 |
| Complement C3 (BS10263) | .645 |
| Alpha-2-macroglobulin (BSI0197) | .636 |
| Complement C5 (BSI0765) | .636 |
| ApoB100 (BSI0040) | .634 |
| Alpha-1B-glycoprotein (BSI0116) | .634 |
| Not known (BSI0023) | .633 |
| ApoB100 (BSI0670) | .630 |
| Serine threonine protein kinase TBK1 (BSI0112) | .629 |
| Not known (BSI0246) | .629 |
| Not known (BSI0348) | .627 |
| Alpha-1-antichymotrypsin (BSI0186) | .626 |
| Complement C3 (BSI0217) | .623 |
| ApoB100 (BSI0323) | .620 |
| Alpha-1B-glycoprotein (BSI0196) | .618 |
| Alpha-2-antiplasmin (BSI0142) | .618 |
| Ceruloplasmin (BSI0203) | .615 |
| Fibrinogen (BSI0251) | .611 |
| Not known (BSI0022) | .605 |
| Alpha-1-B glycoprotein (BSI0198) | .600 |
| Alpha-2-macroglobulin (BSI0172) | .598 |
| D-vitamin binding protein (BSI0257) | .594 |
| Complement C3 (BSI0190) | .590 |
| Alpha-2-macroglobulin (BSI0038) | .589 |

(continued)

| Protein ID (Probe) | AUC |
|---|---|
| Not known (BSI0201) | .587 |
| Alpha-2-macroglobulin (BSI0195) | .584 |
| Apolipoprotein A1 (BSI0179) | .584 |
| ApoB100 (BSI0136) | .582 |
| Complement C3b (shorter form) (BSI0189) | .580 |
| Not known (BSI0660) | .579 |
| Apo B100 (BSI0208) | .579 |
| IgG (BSI0248) | .572 |
| Hemopexin (BSI0002) | .565 |
| Alpha-2-macroglobulin (BSI0095) | .565 |
| Not known (BSI0281) | .564 |
| Not known (BSI0355) | .562 |
| Not known (BSI0177) | .555 |
| Hornerin (BSI0255) | .547 |
| Alpha-2-macroglobulin (BSI0173) | .546 |
| D vitamin binding protein (BSI0185) | .543 |
| Not known (IgG) (BSI0225) | .543 |
| Not known (BSI0314) | .540 |
| ApoB100 (BSI0214) | .537 |
| Not known (BSI0259) | .537 |
| Ceruloplasmin (BSI0099) | .528 |
| Not known (BSI0311) | .521 |
| Complement C3 (BS10242) | .521 |
| Not known (BSI0180) | .518 |
| ApoB100 (BSI0266) | .518 |
| D-vitamin binding protein (BSI0051) | .512 |
| Not known (BSI0181) | .511 |
| IgG Fc Binding protein (BSI0032) | .507 |
| Alpha-2-macroglobulin (BSI0200) | .504 |
| Serine threonine protein kinase TBK1 (BSI0100) | .504 |
| Not known (BSI0191) | .500 |

*Table 4* **AUC for combinations of biomarkers**

| Biomarker combination | Area | Std. Error | 95% Confidence Interval | |
|---|---|---|---|---|
| | | | Lower | Upper |
| Afamin (BSI0268) and Alpha-1 antichymotrypsin (BSI0221) | .906 | .052 | .804 | 1.000 |
| Afamin/Alpha-1 antichymotrypsin ratio* | .875 | .063 | .752 | .998 |

(continued)

| Biomarker combination | Area | Std. Error | 95% Confidence Interval | |
|---|---|---|---|---|
| | | | Lower | Upper |
| Afamin/Alpha-1 antichymotrypsin Ratio, Complement C5 (BSI0782) | .920 | .052 | .819 | 1.000 |
| Afamin/Alpha-1 antichymotrypsin Ratio, Complement C3 (BSI0243) | .889 | .060 | .771 | 1.000 |
| Afamin/Alpha-1 antichymotrypsin Ratio, Alpha-2-macroglobulin (BSI0195) | .906 | .052 | .805 | 1.000 |
| Afamin/Alpha-1 antichymotrypsin Ratio, Serine Threonine Kinase TBK1 (BSI0112) | .892 | .057 | .779 | 1.000 |
| Afamin/Alpha-1 antichymotrypsin Ratio, ApoE4 (presence/absence) | .925 | .042 | .844 | 1.000 |
| Afamin/Alpha-1 antichymotrypsin Ratio, ApoE4 (0,1 or 2 alleles) | .953 | .032 | .891 | 1.000 |
| Afamin/Alpha-1 antichymotrypsin Ratio, ApoB100 (BSI0289) | .875 | .063 | .752 | .998 |
| Afamin/Alpha-1 antichymotrypsin Ratio, Not Known (BSI0183) | .881 | .064 | .756 | 1.000 |
| Afamin/Alpha-1 antichymotrypsin Ratio, Not Known (BSI0279) | .878 | .064 | .753 | 1.000 |
| Afamin/Alpha-1 antichymotrypsin Ratio, Comp C5 (BSI0782), ApoE4 (presence/absence) | .958 | .028 | .903 | 1.000 |
| Afamin/Alpha-1 antichymotrypsin Ratio, Comp C5 (BSI0782), ApoE4 (0,1 or 2 alleles) | .964 | .028 | .910 | 1.000 |
| *For all Afamin/alpha-1 antichymotrypsin ratio combinations the probes BSI0268 and BSI0221 were used | | | | |

## Claims

1. A method of diagnosing or monitoring a person at risk of developing or having Alzheimer's disease (AD) comprising measuring the concentration or relative level of the biomarkers Afamin and Alpha-1 antichymotrypsin and optionally at least one additional biomarker selected from Alpha-2-macroglobulin, Apolipoprotein B100, complement C3, Serine threonine kinase TBK-1, vitamin D binding protein, alpha-1-B glycoprotein, hemopexin, serum albumin, ceruloplasmin, alpha-2-antiplasmin, apolipoprotein A1, complement factor H, IgG, IgG Fe binding protein, hornerin, fibrinogen, and complement C5 in a fluid sample obtained from a person suspected of having or at risk of having AD; and establishing the significance of the concentrations or relative levels.

2. The method of claim 1 wherein the ratio of afamin to alpha-1 antichymotrypsin is calculated.

3. The method according to claim 2 wherein the ratio of afamin to alpha-1 antichymotrypsin is calculated and the concentration or relative level of at least one additional biomarker selected from serine threonine protein kinase TBK1, alpha-2-macroglobulin, Apolipoprotein B100, complement C3, vitamin D binding protein, alpha-1-B glycoprotein, hemopexin, serum albumin, ceruloplasmin, alpha-2- antiplasmin, apolipoprotein A1, complement factor H, IgG, IgG Fe binding protein, hornerin, fibrinogen, and complement C5 is also measured.

4. The method of claim 3 wherein one additional biomarker is serine threonine protein kinase TBK1.

5. The method of claim 3 wherein one additional biomarker is complement C5.

6. The method of claim 3 wherein one additional biomarker is alpha-2 macroglobulin.

7. The method of claim 3 wherein one additional biomarker is Apolipoprotein B100.

8. The method of claim 3 wherein one additional biomarker is complement C3.

9. The method according to any preceding claim which further comprises determining the genotype of at least one of Apolipoprotein E and Glutathione S-Transferase 1 Omega of a person through identification of the nucleic acid sequence encoding the protein in the genome or through determining the form of protein produced in a fluid sample taken from the person.

10. The method of any of the preceding claims further comprising using the measurements obtained in a classification method to calculate the probability of that person having or being at risk of developing AD.

11. The method according to claim 10, wherein the method of classification is at least one of artificial neural networks, logistic regression, decision trees, random forest, support vector machines.

12. The method according to any of the preceding claims wherein the fluid sample is plasma or serum.


**Patentansprüche**

1. Verfahren zur Diagnose oder Überwachung einer Person, die gefährdet ist, Alzheimer-Krankheit (AD) zu entwickeln oder zu haben, das Folgendes umfasst: Messen der Konzentration oder relativen Höhe der Biomarker Afamin und Alpha-1-Antichymotrypsin und optional mindestens eines zusätzlichen Biomarkers, der aus Folgenden ausgewählt ist: Alpha-2-Makroglobulin, Apolipoprotein B 100, Komplement C3, Serin-Threonin-Kinase TBK-1, Vitamin-D-Bindungsprotein, Alpha-1-B-Glycoprotein, Hämopexin, Serumalbumin, Ceruloplasmin, Alpha-2-Antiplasmin, Apolipoprotein A1, Komplementfaktor H, IgG, IgG-Fe-Bindungsprotein, Hornerin, Fibrinogen und Komplement C5, in einer Flüssigkeitsprobe, die von einer Person erhalten wurde, bei der der Verdacht besteht, dass sie AD hat oder gefährdet ist zu haben; und Ermitteln der Signifikanz der Konzentrationen oder relativen Höhen.

2. Verfahren nach Anspruch 1, wobei das Verhältnis von Afamin zu Alpha-1-Antichymotrypsin berechnet wird.

3. Verfahren nach Anspruch 2, wobei das Verhältnis von Afamin zu Alpha-1-Antichymotrypsin berechnet wird und die Konzentration oder relative Höhe von mindestens einem zusätzlichen Biomarker, der aus Folgenden ausgewählt ist: Serin-Threonin-Proteinkinase TBK1, Alpha-2-Makroglobulin, Apolipoprotein B 100, Komplement C3, Vitamin-D-Bindungsprotein, Alpha-1-B-Glycoprotein, Hämopexin, Serumalbumin, Ceruloplasmin, Alpha-2-Antiplasmin, Apolipoprotein A1, Komplementfaktor H, IgG, IgG-Fe-Bindungsprotein, Hornerin, Fibrinogen und Komplement C5, ebenfalls gemessen wird.

4. Verfahren nach Anspruch 3, wobei ein zusätzlicher Biomarker Serin-Threonin-Proteinkinase TBK1 ist.

5. Verfahren nach Anspruch 3, wobei ein zusätzlicher Biomarker Komplement C5 ist.

6. Verfahren nach Anspruch 3, wobei ein zusätzlicher Biomarker Alpha-2-Makroglobulin ist.

7. Verfahren nach Anspruch 3, wobei ein zusätzlicher Biomarker Apolipoprotein B 100 ist.

8. Verfahren nach Anspruch 3, wobei ein zusätzlicher Biomarker Komplement C3 ist.

9. Verfahren nach einem vorstehenden Anspruch, das weiter Folgendes umfasst: Bestimmen des Genotyps von mindestens einem von Apolipoprotein E und Glutathion-S-Transferase 1 Omega von einer Person durch Identifizierung der Nukleinsäuresequenz, die das Protein kodiert, in dem Genom oder durch Bestimmen der Form von erzeugtem Protein in einer Flüssigkeitsprobe, die von der Person entnommen wurde.

10. Verfahren nach einem der vorstehenden Ansprüche, das weiter die Verwendung der erhaltenen Messungen in einem Klassifikationsverfahren umfasst, um die Wahrscheinlichkeit zu berechnen, dass diese Person AD hat oder gefährdet ist zu entwickeln.

11. Verfahren nach Anspruch 10, wobei das Verfahren der Klassifikation mindestens eines von künstlichen neuronalen Netzen, logistischer Regression, Entscheidungsbäumen, Random Forest, Support-Vektor-Maschinen ist.

12. Verfahren nach einem der vorstehenden Ansprüche, wobei die Flüssigkeitsprobe Plasma oder Serum ist.

**Revendications**

1.  Une méthode de diagnostic ou de surveillance d'un sujet à risque de développer ou déjà atteint de la maladie d'Alzheimer (MA) consistant à mesurer la concentration ou le taux relatif des biomarqueurs Afamine et alpha 1-antichymotrypsine et d'éventuellement au moins un autre biomarqueur sélectionné parmi l'alpha-2-macroglobuline, l'apolipoprotéine B100, la protéine C3 du complément, la sérine thréonine kinase TBK-1, la protéine de liaison de la vitamine D, l'alpha 1-B glycoprotéine, l'hémopexine, l'albumine sérique, la céruloplasmine, l'alpha-2 antiplasmine, l'apolipoprotéine A1, le facteur H du complément, l'IgG, la protéine de liaison IgG Fe, l'hornérine, le fibrinogène, et la protéine C5 du complément, dans un échantillon de liquide prélevé sur un sujet soupçonné de présenter ou d'être à risque de développer la MA ; et à établir l'importance de ces concentrations ou niveaux relatifs.

2.  La méthode de la revendication 1 consistant à calculer le taux d'afamine par rapport au taux d'alpha 1-antichymotrypsine.

3.  La méthode selon la revendication 2, consistant à calculer le taux d'afamine par rapport à l'alpha 1-antichymotrypsine ainsi qu'à mesurer la concentration ou le taux relatif d'au moins un autre biomarqueur, sélectionné parmi la sérine thréonine protéine kinase TBK1, l'alpha-2-macroglobuline, l'apolipoprotéine B100, la protéine C3 du complément, de la protéine de liaison de la vitamine D, l'alpha-1-B glycoprotéine, l'hémopexine, l'albumine sérique, la céruloplasmine, l'alpha-2 antiplasmine, l'apolipoprotéine A1, le facteur H du complément, l'IgG, la protéine de liaison IgG Fe, l'hornérine, le fibrinogène, et la protéine C5 du complément.

4.  La méthode de la revendication 3 dans laquelle la sérine thréonine protéine kinase TBK1 est un biomarqueur supplémentaire.

5.  La méthode de la revendication 3 dans laquelle la protéine C5 du complément est un biomarqueur supplémentaire.

6.  La méthode de la revendication 3 dans laquelle l'alpha-2 macroglobuline est un biomarqueur supplémentaire.

7.  La méthode de la revendication 3 dans laquelle l'apolipoprotéine B100 est un biomarqueur supplémentaire.

8.  La méthode de la revendication 3 dans laquelle la protéine C3 du complément est un biomarqueur supplémentaire.

9.  La méthode selon l'une quelconque des revendications précédentes, comprenant en outre la détermination du génotype d'au moins l'apolipoprotéine E ou de la glutathion S-transférase Omega 1 d'un sujet, en identifiant la séquence d'acide nucléique codant la protéine dans le génome, ou en déterminant la forme des protéines produites dans un échantillon de liquide provenant dudit sujet.

10. La méthode de l'une quelconque des revendications précédentes, comprenant en outre d'utiliser les mesures, obtenues dans une méthode de classification, afin de calculer la probabilité, pour ce sujet, de présenter ou d'être à risque de développer la MA.

11. La méthode selon la revendication 10, dans laquelle la méthode de classification consiste au moins en l'une des méthodes de réseau de neurones artificiels, de régression logistique, d'arbres de décision, d'arbres aléatoires, de machines à vecteurs de support.

12. La méthode selon l'une quelconque des revendications précédentes, dans laquelle l'échantillon de fluide consiste en du plasma ou du sérum.

**Figure 1**

**Figure 2**

**Figure 3**

Figure 4

Figure 5

**Figure 6**

**Figure 7**

**Figure 8**

**Figure 9**

Figure 10

Figure 11

**Figure 12**

Afamin/Alpha-1 antichymotrypsin ratio

Figure 13

Figure 14

Afamin/Alpha-1 antichymotrypsin Ratio, Alpha-2 macroglobulin

Figure 15

Figure 16

Afamin/Alpha-1 antichymotrypsin ratio, Complement C5

Figure 17

Figure 18

Figure 19

Afamin/Alpha-1 antichymotrypsin Ratio, Comp C3/C5, ApoE4 (0,1 or 2 alleles)

Figure 20

Control
AD

Tree graph for Group
Num. of non-terminal nodes: 2, Num. of terminal nodes: 3
Model: C&RT

ID=1     N=38
0

APOE

= +ve ApoE4           = -ve ApoE4

ID=2     N=13
AD

ID=3     N=25
Control

Afamin/Alpha-1 antichymotrypsin Ratio

<= 0.930373        > 0.930373

ID=4     N=10
AD

ID=5     N=15
Control

**REFERENCES CITED IN THE DESCRIPTION**

**Patent documents cited in the description**

- EP 2293075 A2
- WO 2011143597 A1
- EP 2293075 A
- EP 2507638 A
- US 20090136966 A
- EP 98307706 A
- EP 98307732 A
- EP 0902394 A
- EP 1227311 A
- EP 1434995 A
- EP 1354623 A